(19) Europäisches Patentamt / European Patent Office / Office européen des brevets

(11) **EP 3 521 497 A1**

(12) **EUROPEAN PATENT APPLICATION**
published in accordance with Art. 153(4) EPC

(43) Date of publication:
**07.08.2019  Bulletin 2019/32**

(21) Application number: **17855902.7**

(22) Date of filing: **20.09.2017**

(51) Int Cl.:
*D04H 3/11* (2012.01)          *A61K 8/02* (2006.01)
*D04H 3/013* (2012.01)

(86) International application number:
**PCT/JP2017/033966**

(87) International publication number:
**WO 2018/061947 (05.04.2018 Gazette 2018/14)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **28.09.2016  JP 2016189590**

(71) Applicant: **ASAHI KASEI KABUSHIKI KAISHA Tokyo 100-0006 (JP)**

(72) Inventors:
• **MACHIOKA, Kyoko**
  **Tokyo 101-8101 (JP)**
• **UME, Shogo**
  **Tokyo 101-8101 (JP)**
• **MASUDA, Chihiro**
  **Tokyo 101-8101 (JP)**

(74) Representative: **dompatent von Kreisler Selting Werner - Partnerschaft von Patent- und Rechtsanwälten mbB Deichmannhaus am Dom Bahnhofsvorplatz 1 50667 Köln (DE)**

(54)  **NONWOVEN FABRIC FOR GEL MASK**

(57)    Provided is a nonwoven fabric for a gel mask having high gel permeability and superior skin adherence and skin contact properties. This nonwoven fabric for a gel mask, which has a plurality of through holes, has the following features: (1) bulk density greater than 0.02 g/cm$^3$ and less than or equal to 0.15 g/cm$^3$; (2) average hole opening area of the through holes of 0.40 mm$^2$ - 1.00 mm$^2$; (3) proportion occupies by through holes in the nonwoven fabric surface of 16 - 50%; and (4) thickness of the nonwoven fabric of 0.10 - 0.40 mm.

EP 3 521 497 A1

**Description**

FIELD

The present invention relates to a non-woven fabric for gel masks having high gel permeability and excellent skin adhesion and skin contact properties.

BACKGROUND

Conventionally, gel masks capable of exhibiting functions such as enhancing the water retention properties of the surface of the skin are used as a pack or paste material for cosmetic purposes. In recent years, for example, Patent Literature 1 described below proposes a gel sheet characterized by having high strength using a woven fabric or a non-woven fabric as a support. As described in Patent Literature 2 below, cellulose non-woven fabrics having good gel retention and transparency when wet have also been developed, though such non-woven fabrics are thin non-woven fabrics.

However, the development of non-woven fabrics for use as gel masks for cosmetic care, which has been growing in demand in recent years, is not satisfactory and has the following problems:

(1) the time required for gel permeation into the support is slow, whereby processing speed cannot be increased;
(2) air bubbles remain in the gel mask, bringing about insufficient drug solution transferability uniformity; and
(3) the adhesiveness of the gel mask is insufficient.

[CITATION LIST]

[PATENT LITERATURE]

[PTL 1] Japanese Unexamined Patent Publication (Kokai) No. 2009-108006
[PTL 2] Japanese Unexamined Patent Publication (Kokai) No. 2016-701

SUMMARY

[TECHNICAL PROBLEM]

In light of the above problems of the prior art, the object of the present invention is to provide a non-woven fabric for gel masks having high gel permeability and excellent skin adhesion and skin contact properties.

[SOLUTION TO PROBLEM]

As a result of rigorous investigation and repeated experimentation in order to achieve the above object, the present inventors have unexpectedly discovered that the above object can be achieved by a non-woven fabric for gel masks in which the bulk density is 0.15 g/cm$^3$ or less, the hole opening area of the through-holes is 0.40 mm$^2$ or more, and the area of the surface of the non-woven fabric occupied by through-holes is 16% or more, and have completed the present invention. Specifically, the present invention is as described below.

[1] A non-woven fabric for a gel mask comprising a plurality of through-holes, wherein the non-woven fabric has the following characteristics:

(1) the bulk density of the non-woven fabric is in the range of greater than 0.02 g/cm$^3$ to 0.15 g/cm$^3$;
(2) the average hole opening area of the through-holes is in the range of 0.40 mm$^2$ to 1.00 2 mm ;
(3) the proportion of a surface of the non-woven fabric occupied by the through-holes is in the range of 16% to 50%; and
(4) the thickness of the non-woven fabric is in the range of 0.10 mm to 0.40 mm.

[2] The non-woven fabric for a gel mask according to [1] having an ventilation resistance in the range of 7 Pa·S/m to 35 Pa·S/m.
[3] The non-woven fabric for a gel mask according to [1] or [2] having a wet 20% modulus in the range of 0.20 N/50 mm to 1.50 N/50 mm.

[4] The non-woven fabric for a gel mask according to any one of [1] to [3], wherein fibers constituting the non-woven fabric are continuous long fibers.

[5] The non-woven fabric for a gel mask according to any one of [1] to [4], wherein 100 wt% of fibers constituting the non-woven fabric are cellulose fibers.

[6] A gel mask comprising the non-woven fabric for a gel mask according to any one of [1] to [5].

ADVANTAGEOUS EFFECTS OF INVENTION

The gel mask using the non-woven fabric of the present invention has a high gel permeability at the time of gel processing, and the obtained gel mask is excellent in skin adhesion and skin contact properties.

DESCRIPTION OF EMBODIMENTS

The embodiments of the present invention will be described in detail below.

The non-woven fabric for gel masks of the present embodiment is a non-woven fabric which is not in a film form and which is not a textile or a knit. A film form is not preferable because gel permeability at the time of gel processing is extremely poor and skin contact is reduced as the face mask hardens. A textile or knit is not preferable because the gel mask may fray along the edges of the support, bringing about a tickling sensation due to contact with the skin when wearing the gel mask and oils are often used during processing, which can be irritating to the skin, which is undesirable from physical and chemical viewpoints.

The fibers used in the present embodiment are not particularly limited. Examples thereof include cellulose, nylon, polyester, and polypropylene fibers. Cellulose fibers are preferable. Examples of cellulose fibers include regenerated cellulose fibers such as cuprammonium rayon, viscose rayon, Tencel (lyocell), and polynosic and natural cellulose fibers such as cotton, pulp, and hemp. Regenerated cellulose fibers are preferable. Though either continuous long fibers or short fibers may be used, continuous long fibers produce less link than short fibers, do not cause a tickling sensation when a gel mask is worn, and are excellent in liquid absorption and strength.

The non-woven fabric of the present embodiment comprises desirably 50 wt% or more of the above cellulose fibers, more preferably 70 wt% or more, and even more preferably 90 wt% or more. When the non-woven fabric comprises less than 50 wt% of cellulose fibers, the gel retention and the liquid absorption rate are not sufficient, and the transparency as the support is also reduced, which is not preferable for a non-woven fabric for gel masks.

Since there is a risk of a reduction of water absorbability and elution of components when the non-woven fabric is imparted with a binder or surfactant, the non-woven fabric for gel masks of the present embodiment preferably does not include a binder. The basis weight of the non-woven fabric for gel masks is preferably in the range of 8 $g/m^2$ to 30 $g/m^2$, more preferably in the range of 10 $g/m^2$ to 25 $g/m^2$, and even more preferably in the range of 10 $g/m^2$ to 20 $g/m^2$. When the basis weight of the non-woven fabric for gel masks is less than 8 $g/m^2$, the fabric becomes excessively thin, whereby handleability is remarkably reduced, which is not practical. Conversely, when the basis weight exceeds 30 $g/m^2$, though handling is easy, transparency is reduced.

The thickness of the non-woven fabric for gel masks of the present embodiment is in the range of 0.10 mm to 0.40 mm, preferably in the range of 0.17 mm to 0.37 mm, and more preferably in the range of 0.19 mm to 0.35 mm. When the thickness of the non-woven fabric for gel masks is less than 0.10 mm, the fabric becomes excessively thin, whereby tearing may occur at the time of gel processing in some cases. Conversely, when the thickness exceeds 0.40 mm, the penetration speed at the time of gel processing decreases, whereby the gel is retained only on a single side of the gel mask, which is not preferable.

The bulk density of the non-woven fabric for gel masks of the present embodiment is in the range of greater than 0.02 $g/cm^3$ to 0.15 $g/cm^3$, preferably in the range of 0.02 $g/cm^3$ to 0.10 $g/cm^3$, and more preferably in the range of 0.04 $g/cm^3$ to 0.08 $g/cm^3$. When the bulk density of the non-woven fabric for gel masks exceeds 0.15 $g/cm^3$, bubbles remain in the gel mask, resulting in insufficient uniformity in drug solution transferability. Conversely, when the bulk density is less than 0.02 $g/cm^3$, the gaps become excessively large, whereby the gel retention ability decreases.

In the non-woven fabric for gel masks of the present embodiment, the average value of the hole opening area of the through-holes is in the range of 0.40 $mm^2$ to 1.00 $mm^2$, preferably in the range of 0.40 $mm^2$ to 0.90 $mm^2$, and more preferably in the range of 0.50 $mm^2$ to 0.90 $mm^2$. "Through-holes" are holes having a portion penetrating linearly from one side of the non-woven fabric through to the other. When the hole opening area of the through-holes of the non-woven fabric for gel masks is less than 0.40 $mm^2$, the penetration rate at the time of gel processing is reduced. Conversely, when the area exceeds 1.00 $mm^2$, since the through-holes become excessively large, the strength of the non-woven fabric decreases, whereby deformation occurs during processing and the gel retention ability decreases.

The proportion of the surface of the non-woven fabric for gel masks of the present embodiment that is occupied by through-holes is in the range of 16% to 50%, preferably in the range of 17% to 50%, and more preferably in the range of 18% to 50%. When holes occupy a proportion of the surface of the non-woven fabric for gel masks which is less than 16%, the proportion occupied by fibers increases, whereby the penetration rate at the time of gel processing is reduced and bubbles remain in the gel mask. Conversely, when the proportion exceeds 50%, the strength as a non-woven fabric is reduced and deformation such as shrinkage in the width direction occurs at the time of gel processing.

The ventilation resistance of the non-woven fabric for gel masks of the present embodiment is preferably in the range of 7 Pa·S/m to 35 Pa·S/m, more preferably in the range of 10 Pa·S/m to 35 Pa·S/m, and even more preferably in the range of 10 Pa·S/m to 33 Pa·S/m. When the ventilation resistance of the non-woven fabric for gel masks exceeds 35 Pa·S/m, the gel penetration rate at the time of gel processing is reduced, which is not preferable. Conversely, when the ventilation resistance is less than 7 Pa·S/m, the gaps become excessively large, and as a result, the gel retention ability is reduced, which is not preferable.

The wet 20% modulus of the non-woven fabric for gel masks of the present embodiment is preferably in the range of 0.20 N/50 mm to 1.50 N/50 mm, more preferably in the range of 0.25 N/50 mm to 1.50 N/50 mm, and even more preferably in the range of 0.30 N/50 mm to 1.00 N/50 mm. When the wet 20% modulus of the non-woven fabric for gel masks is less than 0.20 N/50 mm, shrinkage in the width direction occurs at the time of gel processing, which is not preferable. Conversely, when the wet 20% modulus exceeds 1.50 N/50 mm, the texture of the non-woven fabric becomes hard and the adhesion as a gel mask is reduced, which is not preferable.

The gel used in the non-woven fabric for gel masks of the present embodiment is not particularly limited, and a gel comprising gelatin, Japanese gelatin, agar, collagen, hyaluronic acid, or natural gels (aloe, etc.) can be used. The phrase "gel mask" refers to a face mask made by cooling and hardening a serum (cosmetic solution). The gel, which is in a gel state at ambient temperature, covers the entire surface of the non-woven fabric, is easy to handle, and when in close contact with the skin, liquefies at skin temperature, whereby the chemical permeability to the skin is high. The manufacturing method of the non-woven fabric for gel masks of the present embodiment can be, for example, as follows.

After spinning a spun yarn onto a net and laminating in sheet form, the yarn can be hydroentangled to obtain a drying step. Regarding the characteristics of the non-woven fabric for gel masks of the present embodiment, the number of fibers is reduced by 20% as compared with conventional products and the lower layer net at the time of hydroentanglement is enlarged. By enlarging the through-holes, decreasing the thickness of the fabric, and providing appropriate gaps, the gel can quickly reach the back side of the non-woven fabric without diffusion of the gel, whereby gel processing speed can be increased and a gel mask in which no bubbles remain after gel processing can be obtained. Furthermore, though it was believed that enlarging the through-holes and reducing the number of fibers reduced mechanical properties such as strength, when spinning the yarn onto the net, since the swing width increases by 20%, the number of single yarn contacts increases, and shrinkage in the width direction does not occur at the time of gel processing, whereby it is possible to prevent a reduction in processing speed due to insufficient strength.

The non-woven fabric for gel masks functions as a support for the gel mask.

By increasing the hole opening area of the through-holes of the non-woven fabric and the proportion of through-holes in the surface of the non-woven fabric, when applying the gel to the non-woven fabric, the time necessary for the gel to reach the back side of the fabric can be reduced. Furthermore, it is possible to uniformly disperse the gel on the back side of the non-woven fabric. Additionally, by increasing the proportion of through-holes in the surface of the non-woven fabric, paths for the discharge of air entering at the time of gel processing are formed, whereby few gas bubbles remain in the gel mask. Thus, by obtaining a gel mask having high uniformity, it is possible to apply a chemical liquid component to the skin without irregularities when the gel mask is worn.

Conversely, the portions of the surface of the non-woven fabric which are not through-holes (the portions occupied by fibers) function as a support for the gel mask, and are necessary for maintaining strength. By increasing the proportion of the portions that are not through-holes in the surface of the non-woven fabric, it is possible to prevent shrinkage in the width direction of the support when processing the gel. Furthermore, the handling properties of the gel mask improve as a result of the increase in strength.

Thus, in the present invention, by optimizing the hole opening area of the through-holes of the non-woven fabric and the proportion of through-holes in the surface of the non-woven fabric, all of a prevention of shrinkage of the non-woven fabric in the width direction, an improvement in gel penetration rate, an improvement in the uniformity of gel dispersion, a reduction in air bubbles in the gel mask, and an improvement in handleability as a gel mask can be achieved.

EXAMPLES

The present invention will be more specifically described by way of the Examples below. However, the present invention is not limited only to these Examples in any way. The measurement methods used in the Examples are shown below.

(1) Basis Weight (g/m$^2$)

A gel mask non-woven fabric having an area of 0.05 m$^2$ or more is dried at 105 °C to a constant weight, and is left in a thermostatic chamber at 20 °C and 65% RH for 16 hours or more. Thereafter, the mass is measured, and the mass (g) per m$^2$ of the non-woven fabric is obtained. For this measurement, samples taken from 10 arbitrary locations are measured, and the average value thereof is set as the basis weight (g/m$^2$).

(2) Thickness (mm)

50 points on the non-woven fabric for gel masks are measured under a load of 1.96 kPa in accordance with the JIS-L1096 standard thickness test, and the average value thereof is set as the thickness (mm).

(3) Bulk Density (g/cm$^3$)

The bulk density of the non-woven fabric for gel masks is calculated using the following Formula:

$$\text{Bulk Density (g/cm}^3) = \{\text{Basis Weight (g/m}^2) \text{ / Thickness (mm)}\} \text{ / } 1000$$

(4) Hole Opening Area (mm$^2$)

A non-woven fabric for gel masks, which has been left in a thermostatic chamber at 20 °C and 65% RH for 16 hours or more, is observed under a microscope (40 times magnification), the obtained image is analyzed using Image. JJ, and the area of a single through-hole having an area of 0.15 mm$^2$ or more is measured. For this measurement, through-holes are measured at 10 arbitrary locations, and the average value thereof is set as the hole opening area (mm$^2$).

(5) Proportion of Surface of Non-Woven Fabric Occupied by Through-Holes (%)

A non-woven fabric for gel masks, which has been left in a thermostatic chamber at 20 °C and 65% RH for 16 hours or more, is observed under a microscope and the obtained image is analyzed using Image. JJ. In an area A (mm$^2$) of the non-woven fabric which includes 10 through-holes having an area of 0.15 mm$^2$ or more, the total area B (mm$^2$) of the through-hole portions having an area of 0.15 mm$^2$ or more is measured, and the proportion of the surface of the non-woven fabric occupied by holes is calculated by the following formula. In this measurement, samples taken at 10 arbitrary locations are measured, and the average value thereof is obtained.

$$\text{Proportion of the Surface of the Non-Woven Fabric Occupied by Through-Holes (\%)} = (B \text{ / } A) \text{ x } 100$$

(6) Ventilation Resistance (Pa·S/m)

Four non-woven fabrics for gel masks, which have been left in a thermostatic chamber at 20 °C and 65% RH for 16 hours or more, are stacked and the ventilation resistance thereof is measured using an air permeability tester (KES-F8, manufactured by KatoTech). In this measurement, samples taken at 10 arbitrary locations are measured, and the average value thereof is set as the ventilation resistance (Pa·S/m).

(7) Wet 20% modulus (N/50 mm)

In a wet state (the state in which liquid dripping has ceased while suspended vertically after immersion in pure water for 16 hours), the stress at 20% elongation in the machining direction of the non-woven fabric for gel masks is measured by a tensile test in accordance with JIS-L1096. For this measurement, samples taken at 10 arbitrary locations are measured, and the average value thereof is set as the wet 20% modulus (N/50 mm).

(8) Gel Permeability (sec)

A non-woven fabric for gel masks, which has been left in a thermostatic chamber at 20 °C and 65% RH for 16 hours or more (10 cm square, 1 sample), is placed in the center of the top of a tripod stand (13 cm height) having a hole with a diameter of 5 cm or more, 2 mL of cosmetic liquid (cleansing liquid made from moisturizing cream, manufactured by Shisedo) is dripped (1 mL/sec) from above the non-woven fabric at a distance of 1 cm or less, and the time until the cosmetic liquid passes through the non-woven fabric and drips to the ground is measured. For this measurement, samples taken at 10 arbitrary locations are measured, and the average value thereof is set as the gel permeability (sec).

(9) Confirmation of Bubbles in Gel Mask

The non-woven fabric for gel masks which was left in a thermostatic chamber at 20 °C and 65% RH for 16 hours or more (30 cm square) is coated with a 60 °C liquid having a viscosity of 3000 cps with a knife coater (automatic coating machine (control coater) specification) at a rate of 20 mm/sec. Bubbling of the obtained mask is evaluated in accordance with the following evaluation criteria. Samples are arbitrarily taken at 5 locations, and the average value of the results evaluated in accordance with the following evaluation criteria is obtained.

<Evaluation Criteria>

Excellent: No bubbling
Good: Bubbling is present, but small and inconspicuous
Poor: Significant bubbling present

(10) Comfort as Facemask (points)

Sensory evaluation is carried out with 20 subjects regarding the comfort of the facemask. The evaluation items and evaluation criteria are as follows, and the average value of the 20 subjects is set as the value of the comfort sensory evaluation of the sample.

<Evaluation items>

(1) The mask does not easily peel off after 10 minutes have elapsed and adhesion is high.
(2) There is no discomfort such as tickling while wearing the mask.

<Evaluation Criteria>

3 points: All of the above evaluation items are satisfied.
2 points: One of the above evaluation items is satisfied.
1 point: None of the above evaluation items are satisfied.

[Example 1]

A spinning stock solution was prepared by dissolving cotton linters in a cuprammonium solution (10 wt% cotton linters, 7 wt% ammonia, 3 wt% copper). The spinning stock solution was continuously spun under netting tension on a net in four layers to obtain a web of cellulose continuous long fibers. The obtained web of cellulose continuous long fibers was regenerated with dilute sulfuric acid, and the regenerated web of cellulose continuous long fibers obtained after washing with water was hydroentangled on a 20-mesh conveyor net and dried. The net speed was 45 m/min. Regarding the obtained non-woven fabric, the basis weight was 12.7 $g/m^2$, the thickness was 0.20 mm, and the bulk density was 0.064 $g/cm^3$. The characteristics of the obtained non-woven fabric are shown in Table 1 below, and the evaluation results as a non-woven fabric for gel masks are shown in Table 2 below.

[Example 2]

A regenerated cellulose continuous long fibers non-woven fabric was obtained under the same conditions as Example 1 except that the net speed was 35 m/min. Regarding the obtained non-woven fabric, the basis weight was 16.0 $g/m^2$, the thickness was 0.25 mm, and the bulk density was 0.064 $g/cm^3$. The characteristics of the obtained non-woven fabric are shown in Table 1 below, and the evaluation results as a non-woven fabric for gel masks are shown in Table 2 below.

EP 3 521 497 A1

[Example 3]

A regenerated cellulose continuous long fibers non-woven fabric was obtained under the same conditions as Example 1 except that the net speed was 28 m/min. Regarding the obtained non-woven fabric, the basis weight was 18.8 g/m$^2$, the thickness was 0.30 mm, and the bulk density was 0.063 g/cm$^3$. The characteristics of the obtained non-woven fabric are shown in Table 1 below, and the evaluation results as a non-woven fabric for gel masks are shown in Table 2 below.

[Example 4]

A regenerated cellulose continuous long fibers non-woven fabric was obtained under the same conditions as Example 1 except that the spinning stock solution was continuously spun under netting tension on a net in five layers to obtain a web of cellulose continuous long fibers, and the net speed was 35 m/min. Regarding the obtained non-woven fabric, the basis weight was 19.8 g/m$^2$, the thickness was 0.32 mm, and the bulk density was 0.062 g/cm$^3$. The characteristics of the obtained non-woven fabric are shown in Table 1 below, and the evaluation results as a non-woven fabric for gel masks are shown in Table 2 below.

[Example 5]

A spinning stock solution was prepared by dissolving pulp in an N-methylmorpholine N-oxide (NMMO) solution. The spinning stock solution was continuously spun on a net in five layers by a melt blowing method to obtain a web of cellulose continuous long fibers. The obtained web of cellulose continuous long fibers was regenerated with dilute NMMO, and the regenerated web of cellulose continuous long fibers obtained after washing with water was hydroentangled on a 20-mesh conveyor net and dried. The net speed was 40 m/min. Regarding the obtained non-woven fabric, the basis weight was 21.5 g/m$^2$, the thickness was 0.31 mm, and the bulk density was 0.069 g/cm$^3$. The characteristics of the obtained non-woven fabric are shown in Table 1 below, and the evaluation results as a non-woven fabric for gel masks are shown in Table 2 below.

[Example 6]

A regenerated cellulose continuous long fibers non-woven fabric was obtained under the same conditions as Example 4 except that the net speed was 23 m/min. Regarding the obtained non-woven fabric, the basis weight was 29.5 g/m$^2$, the thickness was 0.21 mm, and the bulk density was 0.141 g/cm$^3$. The characteristics of the obtained non-woven fabric are shown in Table 1 below, and the evaluation results as a non-woven fabric for gel masks are shown in Table 2 below.

[Example 7]

A regenerated cellulose continuous long fibers non-woven fabric was obtained under the same conditions as Example 1 except that the net speed was 58 m/min. Regarding the obtained non-woven fabric, the basis weight was 9.8 g/m$^2$, the thickness was 0.13 mm, and the bulk density was 0.075 g/cm$^3$. The characteristics of the obtained non-woven fabric are shown in Table 1 below, and the evaluation results as a non-woven fabric for gel masks are shown in Table 2 below.

[Example 8]

The non-woven fabric obtained in Example 7 was subjected to high-pressure hydroentangling on a 20-mesh conveyor net and then dried. The net speed was 30 m/min. Regarding the obtained non-woven fabric, the basis weight was 10.4 g/m$^2$, the thickness was 0.31 mm, and the bulk density was 0.034 g/cm$^3$. The characteristics of the obtained non-woven fabric are shown in Table 1 below, and the evaluation results as a non-woven fabric for gel masks are shown in Table 2 below.

[Example 9]

A regenerated cellulose continuous long fibers non-woven fabric was obtained under the same conditions as Example 4 except that the conveyor net was 10-mesh and the net speed was 45 m/min. Regarding the obtained non-woven fabric, the basis weight was 15.2 g/m$^2$, the thickness was 0.30 mm, and the bulk density was 0.051 g/cm$^3$. The characteristics of the obtained non-woven fabric are shown in Table 1 below, and the evaluation results as a non-woven fabric for gel masks are shown in Table 2 below.

[Example 10]

A regenerated cellulose continuous long fibers non-woven fabric was obtained under the same conditions as Example 9 except that the conveyor net was 30-mesh. Regarding the obtained non-woven fabric, the basis weight was 15.2 g/m$^2$, the thickness was 0.25 mm, and the bulk density was 0.061 g/cm$^3$. The characteristics of the obtained non-woven fabric are shown in Table 1 below, and the evaluation results as a non-woven fabric for gel masks are shown in Table 2 below.

[Example 11]

A regenerated cellulose continuous long fibers non-woven fabric was obtained under the same conditions as Example 1 except that the conveyor net was 10-mesh and the net speed was 57 m/min. Regarding the obtained non-woven fabric, the basis weight was 10.1 g/m$^2$, the thickness was 0.18 mm, and the bulk density was 0.056 g/cm$^3$. The characteristics of the obtained non-woven fabric are shown in Table 1 below, and the evaluation results as a non-woven fabric for gel masks are shown in Table 2 below.

[Example 12]

The non-woven fabric obtained in Example 1 and the non-woven fabric obtained in Example 2 were overlaid and pressed using a flat roller to obtain a composite sheet. The obtained composite sheet was subjected to high pressure hydroentangling with a 10-mesh conveyor net and then dried. The net speed was 10 m/min. Regarding the obtained non-woven fabric, the basis weight was 27.8 g/m$^2$, the thickness was 0.27 mm, and the bulk density was 0.103 g/cm$^3$. The characteristics of the obtained non-woven fabric are shown in Table 1 below, and the evaluation results as a non-woven fabric for gel masks are shown in Table 2 below.

[Example 13]

A non-woven fabric of Tencel (lyocell) fibers (fineness 1.7 dtex, fibers length 38 mm, basis weight 27.1 g/m$^2$) was subjected to high-pressure hydroentangling with a 10-mesh conveyor net and then dried. Regarding the obtained non-woven fabric, the basis weight was 25.2 g/m$^2$, the thickness was 0.36 mm, and the bulk density was 0.070 g/cm$^3$. The characteristics of the obtained non-woven fabric are shown in Table 1 below, and the evaluation results as a non-woven fabric for gel masks are shown in Table 2 below.

[Comparative Example 1]

A spinning stock solution was prepared by dissolving cotton linters in a cuprammonium solution (10 wt% cotton linters, 7 wt% ammonia, 3 wt% copper). The spinning stock solution was continuously spun under netting tension on a net in five layers to obtain a web of cellulose continuous long fibers. The obtained web of cellulose continuous long fibers was regenerated with dilute sulfuric acid, and the regenerated web of cellulose continuous long fibers obtained after washing with water was hydroentangled on a 40-mesh conveyor net and dried. The net speed was 35 m/min. Regarding the obtained non-woven fabric, the basis weight was 20.4 g/m$^2$, the thickness was 0.28 mm, and the bulk density was 0.073 g/cm$^3$. The characteristics of the obtained non-woven fabric are shown in Table 1 below, and the evaluation results as a non-woven fabric for gel masks are shown in Table 2 below.

[Comparative Example 2]

A spinning stock solution was prepared by dissolving cotton linters in a cuprammonium solution (10 wt% cotton linters, 7 wt% ammonia, 3 wt% copper). The spinning stock solution was continuously spun under netting tension on a net in five layers to obtain a web of cellulose continuous long fibers. The obtained web of cellulose continuous long fibers was regenerated with dilute sulfuric acid, the regenerated web of cellulose continuous long fibers obtained after washing with water was pass through an 18-mesh upper layer net (the value of the number of meshes per inch was 18), passed through a 10-mesh lower layer net, hydroentangled with a water needle under a pressure of 15 kg/cm$^2$, and dried. The net speed was 48 m/min. Regarding the obtained non-woven fabric, the basis weight was 20.4 g/m$^2$, the thickness was 0.28 mm, and the bulk density was 0.073 g/cm$^3$. The characteristics of the obtained non-woven fabric are shown in Table 1 below, and the evaluation results as a non-woven fabric for gel masks are shown in Table 2 below.

[Comparative Example 3]

Characteristics measurement and evaluation were performed on a commercially available nylon tricot. The basis weight was 16.3 g/m$^2$, the thickness was 0.15 mm, and the bulk density was 0.109 g/cm$^3$. The characteristics of the obtained non-woven fabric are shown in Table 1 below, and the evaluation results as a non-woven fabric for gel masks are shown in Table 2 below.

[Comparative Example 4]

A regenerated cellulose continuous long fibers non-woven fabric was obtained under the same conditions as Comparative Example 1 except that the spinning stock solution was continuously spun under netting tension on a net in four layers to obtain a web of cellulose continuous long fibers, the net speed was 90 m/min, and the conveyor net was 40-mesh. Regarding the obtained non-woven fabric, the basis weight was 7.5 g/m$^2$, the thickness was 0.09 mm, and the bulk density was 0.083 g/cm$^3$. The characteristics of the obtained non-woven fabric are shown in Table 1 below, and the evaluation results as a non-woven fabric for gel masks are shown in Table 2 below.

[Comparative Example 5]

Two of the non-woven fabric obtained in Comparative Example 1 were overlaid and pressed using a flat roller to obtain a composite sheet. The obtained composite sheet was subjected to high-pressure hydroentangling with a 40-mesh conveyor net and then dried. The net speed was 20 m/min. Regarding the obtained non-woven fabric, the basis weight was 39.5 g/m$^2$, the thickness was 0.24 mm, and the bulk density was 0.164 g/cm$^3$. The characteristics of the obtained non-woven fabric are shown in Table 1 below, and the evaluation results as a non-woven fabric for gel masks are shown in Table 2 below.

[Comparative Example 6]

A non-woven fabric of cotton fibers (basis weight 35.1 g/m$^2$) was subjected to high-pressure hydroentangling with a 40-mesh conveyor net and then dried. The net speed was 30 m/min. Regarding the obtained non-woven fabric, the basis weight was 32.3 g/m$^2$, the thickness was 0.43 mm, and the bulk density was 0.075 g/cm$^3$. The characteristics of the obtained non-woven fabric are shown in Table 1 below, and the evaluation results as a non-woven fabric for gel masks are shown in Table 2 below.

[Comparative Example 7]

The non-woven fabric obtained in Comparative Example 6 was subjected to high-pressure hydroentangling with a 10-mesh conveyor net and then dried. The net speed was 10 m/min. Regarding the obtained non-woven fabric, the basis weight was 29.4 g/m$^2$, the thickness was 0.39 mm, and the bulk density was 0.075 g/cm$^3$. The characteristics of the obtained non-woven fabric are shown in Table 1 below, and the evaluation results as a non-woven fabric for gel masks are shown in Table 2 below.

[Comparative Example 8]

The non-woven fabric obtained in Comparative Example 6 was subjected to high-pressure hydroentangling with a 10-mesh conveyor net and then dried. The net speed was 10 m/min. Regarding the obtained non-woven fabric, the basis weight was 25.6 g/m$^2$, the thickness was 0.34 mm, and the bulk density was 0.075 g/cm$^3$. The characteristics of the obtained non-woven fabric are shown in Table 1 below, and the evaluation results as a non-woven fabric for gel masks are shown in Table 2 below.

[Comparative Example 9]

Characteristics measurement and evaluation were performed on a commercially available cotton gauze (cotton tricot). The basis weight was 12.3 g/m$^2$, the thickness was 0.64 mm, and the bulk density was 0.019 g/cm$^3$. The characteristics of the obtained non-woven fabric are shown in Table 1 below, and the evaluation results as a non-woven fabric for gel masks are shown in Table 2 below.

**EP 3 521 497 A1**

[Table 1]

| | Basis Weight (g/m²) | Thickness (mm) | Bulk Density (g/cm³) | Hole Opening Area (mm²) | Proportion of Surface of Non-Woven Fabric Occupied by Through-Holes (%) | Ventilation Resistance (Pa·S/m) | Wet 20% Modulus (N/50 mm) |
|---|---|---|---|---|---|---|---|
| Example 1 | 12.7 | 0.20 | 0.064 | 0.76 | 32.7 | 15.0 | 0.43 |
| Example 2 | 16.0 | 0.25 | 0.064 | 0.69 | 29.5 | 19.6 | 0.53 |
| Example 3 | 18.8 | 0.30 | 0.063 | 0.60 | 24.1 | 26.2 | 0.79 |
| Example 4 | 19.8 | 0.32 | 0.062 | 0.53 | 19.1 | 32.9 | 0.88 |
| Example 5 | 21.5 | 0.31 | 0.069 | 0.65 | 23.7 | 27.0 | 0.91 |
| Example 6 | 29.5 | 0.21 | 0.141 | 0.48 | 20.2 | 34.4 | 0.97 |
| Example 7 | 9.8 | 0.13 | 0.075 | 0.80 | 39.9 | 12.3 | 0.32 |
| Example 8 | 10.4 | 0.31 | 0.034 | 0.81 | 41.2 | 11.6 | 0.28 |
| Example 9 | 15.2 | 0.30 | 0.051 | 0.95 | 38.9 | 12.3 | 0.85 |
| Example 10 | 15.2 | 0.25 | 0.061 | 0.43 | 44.7 | 11.2 | 0.98 |
| Example 11 | 10.1 | 0.18 | 0.056 | 0.89 | 46.4 | 7.5 | 0.24 |
| Example 12 | 27.8 | 0.27 | 0.103 | 0.88 | 32.1 | 13.8 | 1.43 |
| Example 13 | 25.2 | 0.36 | 0.070 | 0.81 | 48.1 | 8.1 | 0.25 |
| Comp. Example 1 | 20.4 | 0.28 | 0.073 | 0.29 | 15.9 | 37.0 | 0.95 |
| Comp. Example 2 | 20.4 | 0.28 | 0.073 | 0.29 | 15.4 | 36.1 | 0.91 |
| Comp. Example 3 | 16.3 | 0.15 | 0.109 | 0.32 | 52.7 | 5.1 | 0.16 |
| Comp. Example 4 | 7.5 | 0.09 | 0.083 | 0.41 | 22.8 | 18.3 | 0.09 |
| Comp. Example 5 | 39.5 | 0.24 | 0.164 | 0.25 | 16.4 | 34.5 | 1.59 |

(continued)

|  | Basis Weight (g/m²) | Thickness (mm) | Bulk Density (g/cm³) | Hole Opening Area (mm²) | Proportion of Surface of Non-Woven Fabric Occupied by Through-Holes (%) | Ventilation Resistance (Pa·S/m) | Wet 20% Modulus (N/ 50 mm) |
|---|---|---|---|---|---|---|---|
| Comp. Example 6 | 32.3 | 0.43 | 0.075 | 0.42 | 14.6 | 42.2 | 1.34 |
| Comp. Example 7 | 29.4 | 0.39 | 0.075 | 0.80 | 14.8 | 34.6 | 0.93 |
| Comp. Example 8 | 25.6 | 0.34 | 0.075 | 1.18 | 48.3 | 4.2 | 0.23 |
| Comp. Example 9 | 12.3 | 0.64 | 0.019 | 0.96 | 62.8 | 2.1 | 0.05 |

[Table 2]

|  | Gel Permeability (sec) | Bubbling | Wearing Comfort as Facemask (pts) |
|---|---|---|---|
| Example 1 | 10 | Excellent | 2.8 |
| Example 2 | 13.7 | Excellent | 2.7 |
| Example 3 | 16.2 | Excellent | 2.7 |
| Example 4 | 18.3 | Good | 2.5 |
| Example 5 | 19.1 | Good | 1.8 |
| Example 6 | 22.8 | Good | 2.4 |
| Example 7 | 6.8 | Excellent | 2.5 |
| Example 8 | 4.1 | Excellent | 2.6 |
| Example 9 | 10.6 | Excellent | 2.4 |
| Example 10 | 15.4 | Good | 2.4 |
| Example 11 | 2.4 | Excellent | 2.1 |
| Example 12 | 9.6 | Excellent | 2.3 |
| Example 13 | 3.5 | Excellent | 1.8 |
| Comp. Example 1 | 25.1 | Poor | 2.1 |
| Comp. Example 2 | 24.3 | Poor | 2.3 |
| Comp. Example 3 | 2.9 | Excellent | 1.2 |
| Comp. Example 4 | 2.4 | Good | 1.5 |
| Comp. Example 5 | 41.2 | Poor | 1.5 |
| Comp. Example 6 | 37.2 | Poor | 1.3 |
| Comp. Example 7 | 32.1 | Poor | 1.3 |
| Comp. Example 8 | 18.1 | Excellent | 1.5 |
| Comp. Example 9 | 2.1 | Excellent | 1.1 |

As shown in Table 2, the gel masks using the non-woven fabric for gel masks of the present embodiment were superior in permeability of at the time of gel processing, a lack of bubbling, and wearing comfort.

INDUSTRIAL APPLICABILITY

The gel mask using the non-woven fabric for gel masks of the present invention has a high gel permeability, and excellent skin adhesion and skin contact properties, and can thus be suitably used as a non-woven fabric for gel masks.

**Claims**

1.  A non-woven fabric for a gel mask comprising a plurality of through-holes, wherein the non-woven fabric has the following characteristics:

    (1) the bulk density of the non-woven fabric is in the range of greater than 0.02 g/cm$^3$ to 0.15 g/cm$^3$;
    (2) the average hole opening area of the through-holes is in the range of 0.40 mm$^2$ to 1.00 mm$^2$;
    (3) the proportion of a surface of the non-woven fabric occupied by the through-holes is in the range of 16% to 50%; and
    (4) the thickness of the non-woven fabric is in the range of 0.10 mm to 0.40 mm.

2.  The non-woven fabric for a gel mask according to claim 1 having an ventilation resistance in the range of 7 Pa·S/m to 35 Pa·S/m.

3.  The non-woven fabric for a gel mask according to claim 1 or 2 having a wet 20% modulus in the range of 0.20 N/50 mm to 1.50 N/50 mm.

4.  The non-woven fabric for a gel mask according to any one of claims 1 to 3, wherein fibers constituting the non-woven fabric are continuous long fibers.

5.  The non-woven fabric for a gel mask according to any one of claims 1 to 4, wherein 100 wt% of fibers constituting the non-woven fabric are cellulose fibers.

6.  A gel mask comprising the non-woven fabric for a gel mask according to any one of claims 1 to 5.

## INTERNATIONAL SEARCH REPORT

| International application No. |
|---|
| PCT/JP2017/033966 |

### A. CLASSIFICATION OF SUBJECT MATTER
*D04H3/11*(2012.01)i, *A61K8/02*(2006.01)i, *D04H3/013*(2012.01)i

According to International Patent Classification (IPC) or to both national classification and IPC

### B. FIELDS SEARCHED

Minimum documentation searched (classification system followed by classification symbols)
D04H3/11, A61K8/02, D04H3/013

Documentation searched other than minimum documentation to the extent that such documents are included in the fields searched
| | | | |
|---|---|---|---|
| Jitsuyo Shinan Koho | 1922-1996 | Jitsuyo Shinan Toroku Koho | 1996-2017 |
| Kokai Jitsuyo Shinan Koho | 1971-2017 | Toroku Jitsuyo Shinan Koho | 1994-2017 |

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| A | JP 2003-292421 A  (Kanebo, Ltd.),<br>15 October 2003 (15.10.2003),<br>(Family: none) | 1-6 |
| A | JP 2016-701 A  (Asahi Kasei Fibers Corp.),<br>07 January 2016 (07.01.2016),<br>(Family: none) | 1-6 |
| A | JP 2906147 B2  (L'Oreal),<br>14 June 1999 (14.06.1999),<br>& US 5026552 A          & EP 309309 A1<br>& DE 3870376 A          & FR 2620914 A<br>& CA 1308663 A          & ES 2008065 A | 1-6 |
| A | JP 4230221 B2  (Sekisui Plastics Co., Ltd.),<br>25 February 2009 (25.02.2009),<br>(Family: none) | 1-6 |

| ☒ | Further documents are listed in the continuation of Box C. | ☐ | See patent family annex. |
|---|---|---|---|

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered    to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 10 November 2017 (10.11.17) | 21 November 2017 (21.11.17) |

| Name and mailing address of the ISA/<br>Japan Patent Office<br>3-4-3,Kasumigaseki,Chiyoda-ku,<br>Tokyo 100-8915,Japan | Authorized officer<br><br>Telephone No. |
|---|---|

Form PCT/ISA/210 (second sheet) (January 2015)

**INTERNATIONAL SEARCH REPORT**

| International application No. |
| --- |
| PCT/JP2017/033966 |

C (Continuation). DOCUMENTS CONSIDERED TO BE RELEVANT

| Category* | Citation of document, with indication, where appropriate, of the relevant passages | Relevant to claim No. |
| --- | --- | --- |
| A | JP 3907872 B2 (Kanebo Cosmetics Inc.), 18 April 2007 (18.04.2007), (Family: none) | 1-6 |
| A | JP 4937603 B2 (Midori Hokuyo Co., Ltd.), 23 May 2012 (23.05.2012), (Family: none) | 1-6 |
| A | JP 5683453 B2 (Lion Corp.), 11 March 2015 (11.03.2015), & WO 2010/113683 A1 & KR 10-2012-0002529 A | 1-6 |
| A | JP 2003-95861 A (Lion Corp.), 03 April 2003 (03.04.2003), (Family: none) | 1-6 |
| A | JP 2005-95495 A (Lion Corp.), 14 April 2005 (14.04.2005), (Family: none) | 1-6 |
| A | WO 2012/132981 A1 (Ozu Corp.), 04 October 2012 (04.10.2012), & CN 103458728 A & TW 201238520 A | 1-6 |

Form PCT/ISA/210 (continuation of second sheet) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2009108006 A

- JP 2016000701 A